# EUROPEAN PATENT APPLICATION

(11) **EP 3 815 738 A1**
(43) Date of publication of application: **05.05.2021**
(21) Application number: 20203474.0
(22) Date of filing: 23.10.2020
(51) Int. Cl.: A61N 1/05, A61N 1/375

(54) **IMPLANTABLE MEDICAL DEVICE COMPRISING AN ANCHORING ELEMENT**

(30) Priority: 01.11.2019 EP 19206698
(71) Applicant: BIOTRONIK SE & Co. KG, 12359 Berlin (DE)
(72) Inventor: DÖRR, Thomas, 12437 Berlin (DE); KOLBERG, Gernot, 12161 Berlin (DE)
(74) Representative: Krämer, Thomas

(57) **Abstract**

An implantable medical device (1) for implantation into a patient comprises a body (100, 150) and an anchoring element (13) for anchoring the body (100, 150) to tissue at a location of interest, the anchoring element (13) being arranged on the body (100, 150) and having an electrode arrangement for at least on of emitting an electrical stimulation signal and sensing an electrical sense signal. The electrode arrangement comprises a multiplicity of electrodes (132A-132D) arranged at different locations on the anchoring element (13).

## Description

The present invention relates to an implantable medical device for implantation into a patient according to the preamble of claim 1.

An implantable medical device of this kind comprises a body, and an anchoring element for anchoring the body to tissue at a location of interest, the anchoring element being arranged on the body and having an electrode arrangement for at least on of emitting an electrical stimulation signal and sensing an electrical sense signal.

The implantable medical device may for example have the shape of a leadless stimulation device, such as a leadless pacemaker device. In this case the body is formed by the housing of the leadless pacemaker device, which encapsulates components of the leadless pacemaker device such as a processor, a data memory, a battery and other processing equipment to allow for operation of the leadless pacemaker device in an autarkic manner. The leadless pacemaker device may be implanted directly into the heart and may operate within the heart, for example within the right ventricle of the heart, without requiring any leads for placing an electrode at a location of interest within the heart.

Alternatively, the implantable medical device may be a stimulation device which comprises a generator to be implanted for example subcutaneously at a location remote from the heart. In this case the body is formed for example by a lead extending from the generator into the heart to allow for a stimulation or a sensing of signals at a location of interest within the heart, for example within the right ventricle.

With common electrode arrangements of implantable medical devices, an injection of stimulation signals generally is possible at the surface of intra-cardiac tissue, an electrode being in contact with intra-cardiac tissue in order to allow a injection of stimulation energy into the tissue. With new approaches for example for providing a stimulation in case of a so-called left bundle block, it may be desired to provide for an excitation in a localized fashion in the region of the so-called left bundle branch, which requires to engage with intra-cardiac tissue in the range of the septum of the heart and to place an electrode in the vicinity of the left bundle branch, such that stimulation energy may be specifically injected into the left bundle branch. As this requires a piercing of the septum, there is a general desire to provide for an anchoring of an implantable medical device on intra-cardiac tissue which is easy to establish and allows for a spatially differentiated excitation of tissue, in particular in the context of a left bundle branch pacing.

In particular, when introducing an electrode for example arranged on a lead from the right ventricle into the septum in order to reach towards the left bundle branch, the electrode must be inserted into the tissue to reach a substantial depth in order to come to lie in the vicinity of the left bundle branch. This comes with the inherent risk that a piercing structure on which the electrode is arranged may pierce through the septum and may reach into the left ventricle. In addition, the piercing itself possibly may have a significant impact on tissue and may even destroy tissue. In addition, when placing an electrode on the septum to reach to the left bundle branch, there is a risk of dislocation, for example when the electrode is arranged on an anchoring device in the shape of a screw, such that an electrical coupling in between the electrode and the left bundle branch may deteriorate over time due to for example a displacement of the screw, potentially leading to a capture loss.

WO 2008/058265 A2 discloses a cardiac stimulation system and method which allow to deliver a left ventricle stimulator from a right ventricle lead system in the right ventricle chamber, into a right side of the septum at a first location, and transmuscularly from the first location to a second location along the left side of the septum. The left ventricle stimulator is fixed at the second location for transmuscular stimulation of the left ventricular conduction system. A biventricular simulation system further includes a right ventricle stimulator also delivered by the right ventricle lead system to the first location along the right side of the septum for right ventricular stimulation.

US 2009/0276000 A1 discloses a method for delivering physiological pacing by selecting an electrode implant site for sensing cardiac signals, which is in proximity to the hearts intrinsic conduction system. An arrangement of multiple electrodes herein is arranged on a tip of a lead.

US 10,406,370 discloses a device for providing cardiac pacing by multiple electrodes inserted using a single conduit. Acceptable electrodes herein are selected as active based on a predetermined criteria, and cardiac stimulation is provided for multiple chambers of the heart from a single location.

It is an object of the instant invention to provide an implantable medical device which allows for an easy implantation and operation, in particular in order to provide for a left bundle branch pacing operation.

This object is achieved for means of an implantable medical device comprising the features of claim 1.

Accordingly, the electrode arrangement comprises a multiplicity of electrodes arranged at different locations on the anchoring element.

The body may extend longitudinally along a longitudinal axis. The body herein may for example form a distal end to be placed on tissue upon implantation of the implantable medical device, the anchoring element being arranged on and extending from the distal end. In particular, the anchoring element may extend from the body generally along the longitudinal axis, the anchoring element protruding from the body in order to allow a piercing of tissue by means of the anchoring element in order to provide for an anchoring of the implantable medical device on tissue.

In one embodiment, the body may be formed by a lead which is connectable to a generator of the implantable medical device. In this case, the generator may be implanted into a patient for example subcutaneously remote from the heart, the lead forming the body extending from the generator into the heart such that the body with the anchoring element and the electrodes arranged thereon is placed in the heart, for example within the right ventricle in order to engage with tissue at the right ventricle. If the anchoring element is placed at the distal end of the body, the distal end is to be implanted into the heart to engage with intra-cardiac tissue for anchoring the body with its distal end on tissue within the heart. By engaging with tissue, herein, the electrodes arranged on the anchoring element engage with tissue and hence may be used to at least one of emitting an electrical stimulation signal and sensing an electrical sense signal.

In another embodiment, the body may be formed by a housing of a leadless pacemaker device. In this case, the implantable medical device is formed as a leadless device, which does not comprise leads extending from a location outside of the heart into the heart for providing for a stimulation and/or sensing within the heart. The housing of the leadless pacemaker device may be placed on tissue with a distal end formed by the housing, the anchoring element with the electrodes arranged thereon beneficially being placed on the distal end and engaging with tissue when placing the leadless pacemaker device on tissue with its distal end.

In one embodiment, the anchoring element extends from the body along an axial length of extension, wherein the electrodes are axially displaced with respect to one another along the axial length of extension. The anchoring element herein may extend linearly from the body, may have a coil shape in that the anchoring element for example is wound helically about the longitudinal axis, or may generally be curved, for example in the shape of a tine. The anchoring element hence may extend linearly or may assume a curved shape, the axial length of extension being measured by axially following the straight or curved extension of the anchoring element.

In that the electrodes are displaced axially with respect to one another along the axial length of extension, the electrodes are placed at different locations on the anchoring element. The electrodes may be electrically independent of one another. When the anchoring element pierces into tissue in order to provide for an anchoring of the body of the implantable medical device to tissue, the electrodes engage with tissue and electrically contact the tissue at different locations, such that a stimulation and/or sensing by means of the electrodes may be achieved at different locations along the anchoring element.

In addition or alternatively to an axial displacement of the electrodes, the electrodes may be arranged on different sides with respect to a transverse direction transverse to the axial length of extension. When viewed in a transverse direction with respect to the axial direction of extension, electrodes may for example be placed at diametrically opposite sides with respect to one another, such that the electrodes may face in different directions and hence may emit stimulation signals into and/or sense electrical signals from different directions.

In one embodiment, some or all of the electrodes are arranged at different heights with respect to the body. The anchoring element may for example generally extend from the distal end of the body, the body being configured to be placed on tissue with its distal end. The height at which a particular electrode is arranged with respect to the body herein may be measured along the longitudinal axis along which the body generally extends. By arranging electrodes at different locations on the anchoring element the electrodes may be placed at different heights within tissue when the body is implanted and anchored at a location of interest. By means of the different electrodes hence a stimulation and/or sensing at different locations, in particular at different heights within the tissue may be achieved.

Because the electrodes are placed at different locations on the anchoring element and hence e.g. may be placed within different depths within tissue, using the electrodes an excitation at different locations within the tissue may be provided. The implantable medical device herein may comprise one or more anchoring elements, each carrying multiple electrodes. By selecting one or multiple of the electrodes for at least one of emitting an electrical stimulation signal and sensing an electrical sense signal, an excitation or sensing for example at the left bundle branch in a spatially differentiated manner may be achieved, in that one or multiple electrodes may be selected which are closest and best coupled to the left bundle branch.

Because the implantable medical device may be implanted into the patient's heart such that at least one of the electrodes of the (at least one) anchoring element comes to rest in the region of the left bundle branch and may electrically contact the left bundle branch, a pacing at the left bundle branch may be provided, allowing for a physiological stimulation by achieving a propagation of stimulation signals along the bundle branches of the ventricles at low stimulation thresholds. A left bundle branch stimulation may allow for an easy and reliable implantation and easy stimulation algorithms, in particular not requiring a particular adjustment of AV delays as necessary for example for an HIS bundle pacing.

Implantation of the implantable medical device in particular becomes easy because for the implantation it is not required to place the (at least one) anchoring element in the immediate vicinity of the left bundle branch, in particular at a specific depth. It may be sufficient to generally implant the implantable medical device such that the body of the lead or the leadless pacemaker device is placed on the septum in the general region of the left bundle branch, wherein after implantation and during operation one or multiple electrodes of the (at least one) anchoring element may be selected in order to establish a spatially differentiated, effective coupling to the left bundle branch. Hence, implantation may be easy, and operation may be reliable.

In addition, even when a dislocation of the anchoring element occurs, this may not have an impact on the operation of the implantable medical device, as a different set of electrodes may be used for excitation or sensing in case of a capture loss, such that operation may be modified and adapted over the operative lifespan of the implantable medical device.

In one embodiment, the anchoring element is formed by a helically wound coil element. The anchoring element hence has the shape of a screw which may be screwed into tissue in order to establish an anchoring of the body of the implantable medical device to tissue. The anchoring element extends along a length of extension, wherein multiple electrodes are arranged at different locations on the anchoring element and hence may come into contact with tissue at different locations along the anchoring element.

In another embodiment, the anchoring element is formed by a linearly extending spike element. The spike element for example may extend from the distal end of the body along the longitudinal axis, along which the body generally extends. The spike element may pierce into tissue, such that the spike element comes into contact with tissue and provides for an anchoring of the body with respect to the tissue.

In yet another embodiment, the anchoring element is formed by a flexibly bendable tine. A tine of this kind may in particular be arranged on the body with a first end and may extend from the body in a distal direction to form an apex and to bend backwards generally opposite to the distal direction to form a hook. When being placed on tissue, the tine may engage with tissue such that the apex is placed within tissue, a second, far end of the tine for example being placed outside of the tissue such that the tine forms a hook to anchor the implantable medical device to tissue at a location of interest. By flexibly bending the tine each anchoring element may be brought into engagement with tissue, wherein upon deployment each tine flexibly deforms and resets towards its original, unbent shape in order to form a hook for anchoring the implantable medical device to the tissue.

In one embodiment, the anchoring element may comprise additional, secondary anchoring members, such as a micro-structure in order to provide for an anchoring to tissue. For example when the anchoring element is formed by a linearly extending spike element on which multiple electrodes are placed, secondary anchoring members such as microstructures for example in the shape of micro-tines may be arranged at the outside of the spike element in order to engage with tissue in order to provide for an axial fixation of the anchoring element within tissue and to hence anchor the body of the implantable medical device to the tissue.

The electrodes of the anchoring element, in one embodiment, are electrically independent of one another. Hence, the electrodes may be independently controlled in order to provide for a stimulation and/or sensing by means of one or multiple of the electrodes. In particular, for providing for a spatially differentiated excitation or sensing one or multiple electrodes may be selected which may provide for an effective coupling to tissue at a specific location of interest, for example in the region of the left bundle branch.

In order to form electrically independent electrodes, the anchoring element may have a multilayered shape. For example, the anchoring element may comprise a first electrically conductive layer and a second electrically conductive layer, wherein the first electrically conductive layer and the second electrically conductive layer are electrically separated from one another by an intermediate, electrically insulating layer. One of the electrically conductive layers may herein be arranged radially outwards of the other electrically conductive layer, such that the layers are separated from one another and are electrically independent.

One or multiple of the conductive layers may for example be shaped from a metal material, such as a nickel titanium alloy material, such as nitinol.

In another embodiment, the anchoring element may have an element body formed from a (electrically non-conductive) plastics material, wherein the electrodes may be placed on an outer surface of the element body. Conductors herein may be embedded in the element body to electrically contact the electrodes on the outside of the element body.

In yet another embodiment, the anchoring element may have the shape of a flexible circuit board, with electrodes being formed on the flexible circuit board. The flexible circuit board may be formed from an electrically non-conductive base material, such as a liquid crystal polymer (LCP) material. Conductors, which serve to couple to the electrodes arranged on the flexible circuit board, may be embedded into the flexible circuit board. A wire structure, for example made of nitinol wires, may be embedded into the flexible circuit board in order to mechanically stabilize the flexible circuit board.

In one embodiment, the implantable medical device comprises a processing device configured to control operation of the electrodes for at least one of emitting an electrical stimulation signal and sensing an electrical sense signal using at least one of the electrodes.

The processing device may be part of a generator, in case the body is formed by a lead extending from the generator. Alternatively, the processing device may be part of a leadless pacemaker device forming the implantable medical device. The processing device may control operation of the implantable medical device, in that for example one or multiple electrodes are selected in order to capture the left bundle branch and to provide for a stimulation or sensing at the left bundle branch using one or multiple electrodes of one or multiple anchoring elements.

In one embodiment, the body comprises a further electrode placed at a distance from the anchoring element. The further electrode in particular may be configured to not engage with tissue when implanting the body at a location of interest. The further electrode hence may be configured such that it, upon implantation, remains outside of the tissue. The further electrode may for example have the shape of a ring electrode or a helically wound coil electrode and may for example extend about the longitudinal axis on the body. The further electrode may serve as a counter-electrode for the electrodes arranged on the anchoring element. In addition or alternatively, the further electrode may serve to provide for a different therapy function, for example a shock (defibrillation) function.

The idea of the invention shall subsequently be described in more detail with reference to the embodiments shown in the figures. Herein:
- Fig. 1: shows a schematic view of the human heart, including the Sinotrial node, the Atrioventricular node, the HIS bundle and the left bundle branch and right bundle branch extending from the HIS bundle;
- Fig. 2: shows a schematic drawing of the heart with a lead implanted therein;
- Fig. 3: shows a schematic drawing of the heart with a leadless stimulation device implanted therein;
- Fig. 4: shows a schematic drawing of an implantable medical device having an anchoring element for anchoring the implantable medical device on tissue;
- Fig. 5: shows a schematic drawing of another embodiment of an implantable medical device;
- Fig. 6: shows a view of an anchoring element in the shape of a helically wound coil;
- Fig. 7: shows a view of another embodiment of an anchoring element in the shape of a spike element; and
- Fig. 8: shows a schematic view of an implantable medical device having anchoring elements in the shape of tines.

Subsequently, embodiments of the invention shall be described in detail with reference to the drawings. In the drawings, like reference numerals designate like structural elements.

It is to be noted that the embodiments are not limiting for the invention, but merely represent illustrative examples.

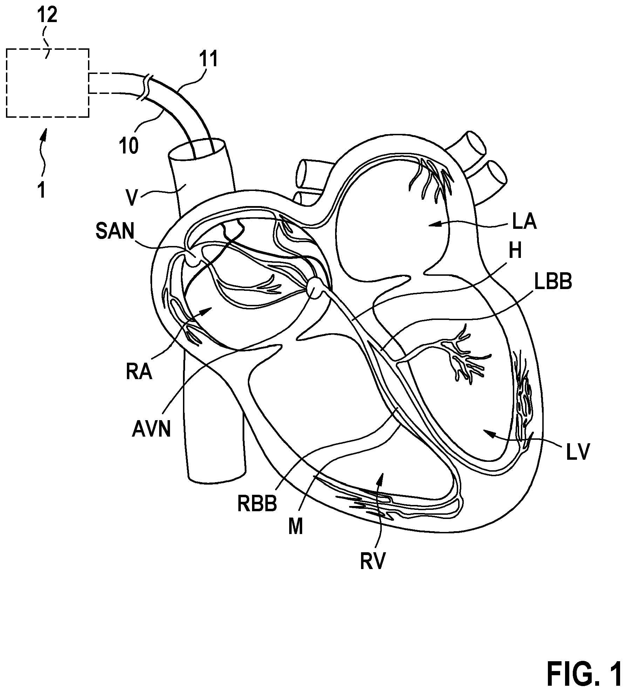

Fig. 1 shows, in a schematic drawing, the human heart comprising the right atrium RA, the right ventricle RV, the left atrium LA and the left ventricle LV, the so-called sinoatrial node SAN being located in the wall of the right atrium RA, the sinoatrial node SAN being formed by a group of cells having the ability to spontaneously produce an electrical impulse that travels through the heart's electrical conduction system, thus causing the heart to contract in order to pump blood through the heart. The atrioventricular node AVN serves to coordinate electrical conduction in between the atria and the ventricles and is located at the lower back section of the intra-atrial septum near the opening of the coronary sinus. From the atrioventricular node AVN the so-called HIS bundle H is extending, the HIS bundle H being comprised of heart muscle cells specialized for electrical conduction and forming part of the electrical conduction system for transmitting electrical impulses from the atrioventricular node AVN via the so-called right bundle branch RBB around the right ventricle RV and via the left bundle branch LBB around the left ventricle LV.

In the embodiment of Fig. 1, an implantable medical device 1 in the shape of a stimulation device, such as a CRT device, is implanted in a patient, the implantable medical device 1 comprising a generator 12 connected to leads 10, 11 extending from the generator 12 through the superior vena V into the patient's heart. By means of the leads 10, 11, electrical signals for providing a pacing action in the heart shall be injected into intra-cardiac tissue potentially at different locations within the heart, and sense signals may be received. In addition, a defibrillation therapy may be performed by an electrode arrangement arranged on one or both of the leads 10, 11.

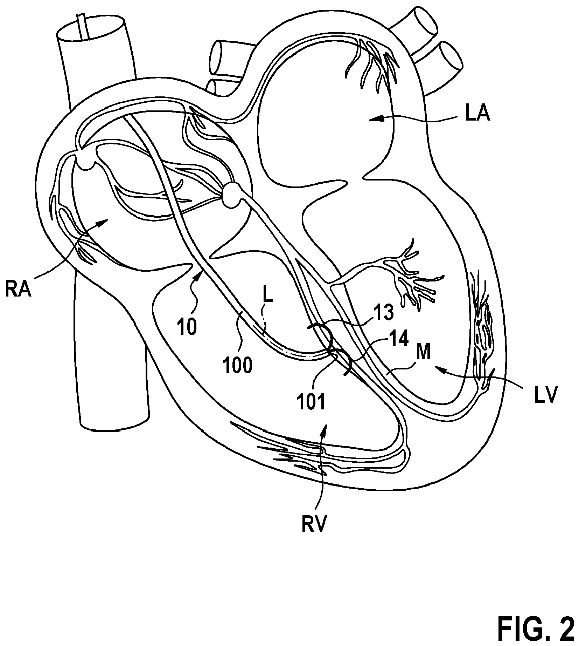

In an embodiment shown in Fig. 2, a lead 10 is implanted into the heart such that it extends into the right ventricle RV of the heart and, at a distal end 101 of a lead body 100, is arranged on intra-cardiac tissue at the septum M in between the right ventricle RV and the left ventricle LV of the heart. At the distal end 101 herein anchoring elements 13, 14 in the shape of tines are arranged, the anchoring elements 13, 14 serving to anchor the lead 10 with its body 100 to tissue in particular in the region of the septum M within the heart. In addition, electrodes are placed on at least one the anchoring elements 13, 14, as shall be described in further detail according to embodiments below.

An implantable medical device 1 as concerned herein may generally be a cardiac stimulation device such as a cardiac pacemaker device. A stimulation device of this kind may comprise a generator 12, as shown in Fig. 1, which may be subcutaneously implanted into a patient at a location remote from the heart, one or multiple leads 10, 11 extending from the generator 12 into the heart for emitting stimulation signals in the heart or for obtaining sense signals at one or multiple locations from the heart.

If the implantable medical device 1 is a stimulation device using leads, a lead 10 forms a generally longitudinal, tubular body 100 extending along a longitudinal axis L, as shown in Fig. 2, which reaches into the heart and is anchored at a location of interest for example on the septum M of the heart in the region of the right ventricle RV.

In another embodiment, the implantable medical device 1 may be a leadless pacemaker device, which does not comprise leads, but has the shape of a capsule and may be directly implanted into the heart, for example into the right ventricle RV of the heart.

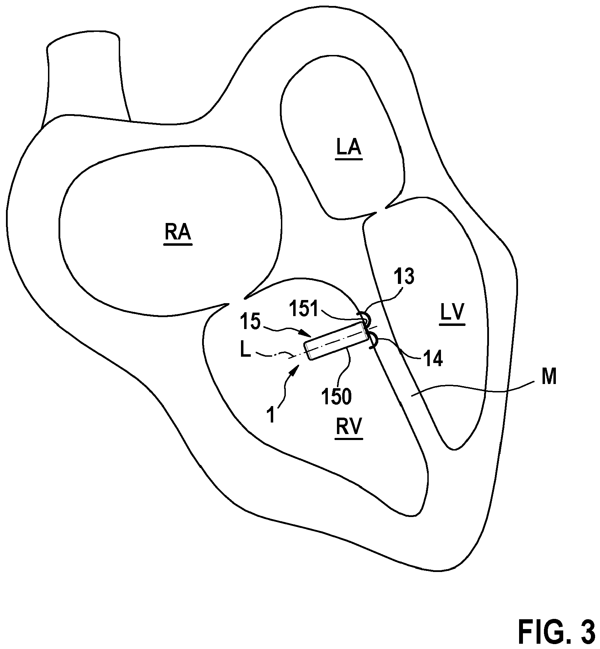

Referring now to Fig. 3, in one embodiment an implantable medical device 1 in the shape of a leadless pacemaker device 15 comprises a body 150 in the shape of a housing which extends longitudinally along a longitudinal axis L and encapsulates components of the leadless pacemaker device 15, such as a processing device, a data memory, a battery, pulse generation circuitry and the like to allow for a stimulation operation immediately within the heart.

In the embodiment of Fig. 3, the body 150 in the shape of the housing of the leadless pacemaker device 15 forms a distal end 151 which is placed on intra-cardiac tissue in the region of the septum M of the heart, anchoring elements 13, 14 being arranged on the body 150 in the region of the distal end 151 and extending from the distal end 151 generally along the longitudinal axis L.

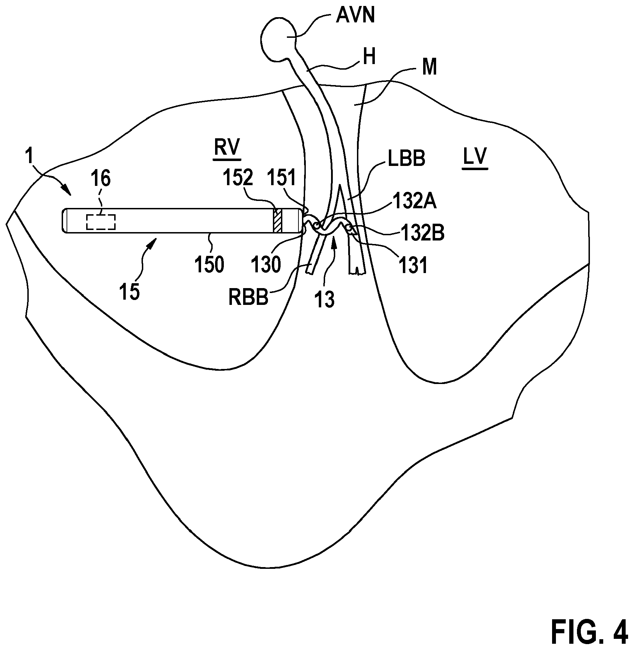

Referring now to Fig. 4, in one embodiment the implantable medical device 1 in the shape of e.g. a leadless pacemaker device 15 comprises an anchoring element 13 in the shape of a helically wound coil, which functions as a screw and may be screwed into tissue in particular in the region of the septum M of the heart. The anchoring element 13, in an implanted state, reaches into the septum M such that electrodes 132A, 132B arranged on the anchoring element 13 may come into electrical contact with the tissue and hence may provide for an excitation and/or sensing within the tissue.

The anchoring element 13 in the shape of the helically wound coil at one end 130 is connected to the distal end 151 of the body 150 and with a far end 131 is placed within tissue at a substantial depth within the tissue. The electrodes 132A, 132B herein are displaced along the length of the anchoring element 13 and hence are placed at different locations within the tissue.

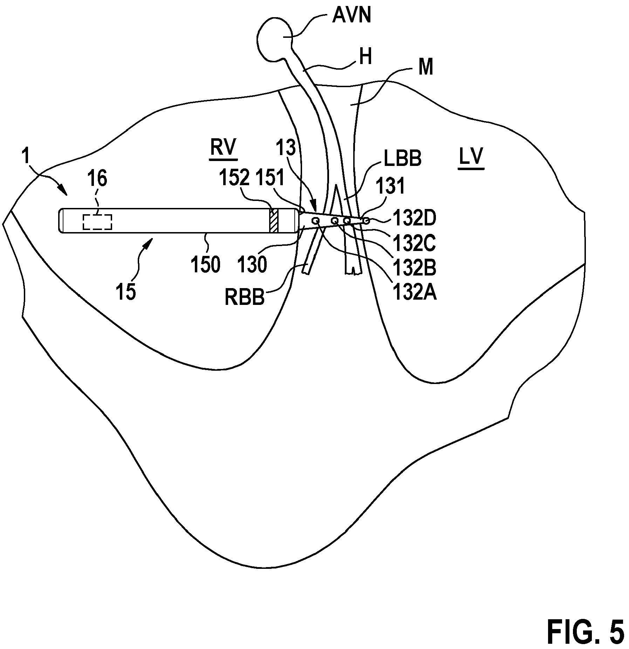

In another embodiment, shown in Fig. 5, the anchoring element 13 is formed by a spike element which linearly extends from the distal end 151 of the body 150 of the implantable medical device 1 which, in the shown embodiment, has the shape of a leadless pacemaker device 15. The anchoring element 13 in the shape of the spike element at one end 130 is connected to the distal end 151 of the body 150 and reaches, with a far, pointed end 131 into the tissue. Electrodes 132A-132D are arranged on the anchoring element 13 and are displaced with respect to one another along the linear length of the anchoring element 13 in the shape of the spike element.

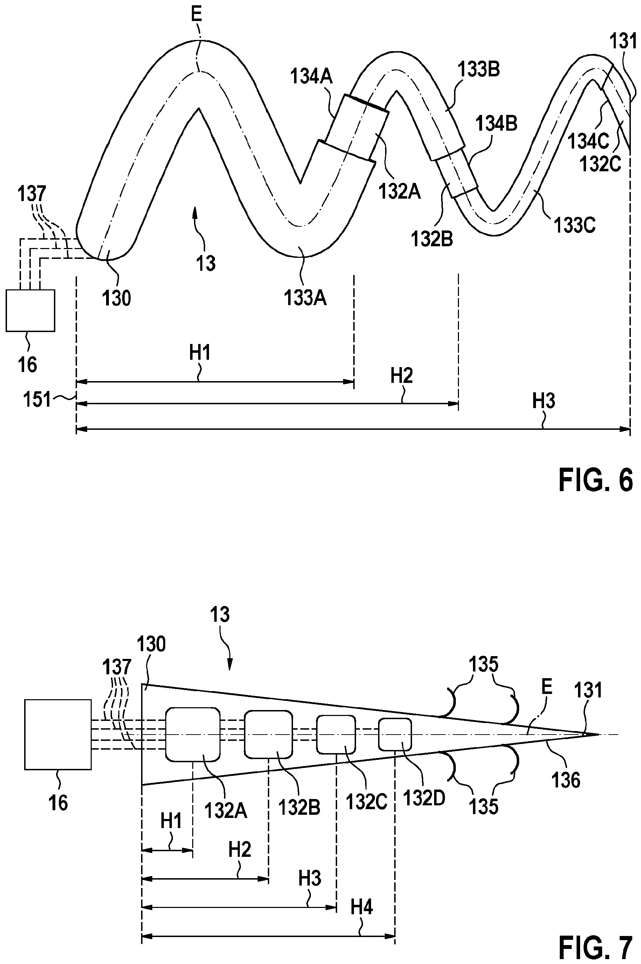

Referring now to Fig. 6, in one embodiment the anchoring element 13, when e.g. being formed as a helically wound coil element having the function of a screw, may have a multilayered structure. In particular, the anchoring element 13 may comprise a multiplicity of electrically conductive layers 134A-134C, which alternate with electrically insulating layers 133A-133C.

In particular, an outer electrically insulating layer 133A extends outside of and covers an electrically conductive layer 134A extending inside the electrically insulating layer 133A, wherein an end of the electrically conductive layer 134A is exposed towards the outside and hence may contact with tissue, the exposed end of the electrically conductive layer 134A hence forming an electrode 132A.

Inside of the electrically conductive layer 134A an electrically insulating layer 133B is formed, inside of which another electrically conductive layer 134B extends. The insulating layer 133B electrically insulates the conductive layer 134A with respect to the conductive layer 134B, wherein an end of the conductive layer 134B is exposed to the outside and hence may come into contact with tissue, the exposed end of the conductive layer 134B hence forming an electrode 132B.

Inside of the conductive layer 134B an insulating layer 133C extends, inside of which an inner conductive core 134C is formed, the inner core 134C being exposed with an end from the insulating layer 133C, the exposed end (which corresponds to the end 131 of the anchoring element 13) forming an electrode 132C.

The electrodes 132A-132C are displaced with respect to one another along the axial length of extension E of the anchoring element 13 and are placed at different heights HI, H2, H3 with respect to the distal end 151 of the body 150 of the implantable medical device 1. The electrodes 132A-132C hence are placed, when implanted in tissue, at different depths within the tissue.

The electrodes 132A-132C are electrically independent of one another and, by means of conductors 137, are connected to a processing device 16 of the implantable medical device 1, which in the case of a leadless pacemaker device 15 is encapsulated in the body 150 formed by the housing of the leadless pacemaker device 15 and which, in case of a stimulation device comprising leads 10, 11, is placed in the generator 12 (see Fig. 1).

In another embodiment, the anchoring element 13 may be formed by a non-conductive (plastics) element body, wherein one or multiple electrodes 132A-132C may be placed on the element body of the anchoring element 13 and may be electrically coupled for example by means of electrical conductors being embedded in the element body.

In yet another embodiment, the anchoring element 13 may for example be formed from a flexible circuit board, formed for example from a liquid crystal polymer material (LCP). In order to provide for a mechanical stabilization, herein, one or multiple metallic wires, such as nitinol wires, may be embedded in the circuit board.

Fig. 7 shows an embodiment of an anchoring element 13 in the shape of a spike element which extends along a straight, linear length of extension E and has a pointed, far end 131. In this embodiment, the anchoring element 13 is formed with an element body 136 for example made of an electrically non-conductive material, such as a plastics material. Electrodes 132A-132D are placed on the element body 136 and, by means of conductors 137, are independently connected to a processing device 16, such that the electrodes 132A-132D may be independently controlled.

Again, the electrodes 132A-132D are placed at different locations on the anchoring element 13 and in particular are arranged at different heights H1-H4 on the element body 136, such that the electrodes 132A-132D are placed at different depths when the anchoring element 13 engages with tissue in an implanted state of the implantable medical device 1.

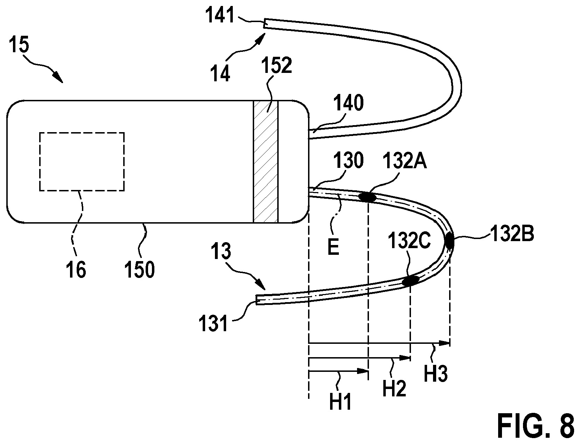

Referring now to Fig. 8, in one embodiment anchoring elements 13, 14 are formed by flexibly bendable tines which at one end 130, 140 each are connected to the body 150 of a leadless pacemaker device 15 to extend from the body 150 to form an apex and to then bend backwards to exit from tissue with far ends 131, 141. The anchoring elements 13, 14 hence form hooks which provide for an anchoring of the implantable medical device 1 on tissue, in that the anchoring elements 13, 14 extend through the tissue and, by forming hooks, fix the implantable medical device 1 to the tissue. Electrodes 132A-132C are placed on at least one of the anchoring elements 13, 14, the electrodes 132A-132C being placed at different locations along the axial length of extension E of the respective anchoring element 13 such that the electrodes 132A-132C come to rest at different heights HI, H2, H3 when the implantable medical device 1 is implanted.

In order to provide electrodes 132A-132C which are electrically independent of each other, the tine 13 may have a multilayered configuration, wherein each electrode 132A-132C is associated with a particular, electrically conductive layer, which is electrically separated from another electrically conductive layer by an intermediate, insulating layer, similarly as described above for the embodiment of Fig. 6.

In the embodiments of Figs. 4 to 8, multiple electrodes 132A-132D are placed at different locations on an anchoring element 13. The electrodes 132A-132D may be electrically independent of one another and may be used in order to emit electrical stimulation signals into and/or receive electrical sense signals from tissue with which the electrodes 132A-132D are in electrical contact.

This may be used, as indicated in Figs. 4 and 5, in particular to provide for a pacing at the left bundle branch LBB. In particular, one of the electrodes 132A-132D may be located in the vicinity of the left bundle branch LBB, such that the respective electrode 132A-132D of the anchoring element 13 may couple with the left bundle branch LBB and hence may capture the left bundle branch LBB to inject signals into the left bundle branch LBB and/or receive sense signals from the left bundle branch LBB.

As different electrodes 132A-132D may be placed at different depths within tissue and hence may provide for an excitation and/or sensing at different depths within tissue, one or multiple electrodes 132A-132D may be selected during operation of the implantable medical device 1 in order to provide for a pacing and/or sensing action. In particular, using a processing device 16 algorithms for operating the implantable medical device 1 may be carried out which may include a configuration algorithm by which one or multiple electrodes 132A-132D are selected which may provide for an optimum coupling to the left bundle branch LBB, such that an effective stimulation and/or sensing at the left bundle branch LBB may be achieved.

Because electrodes 132A-132D may be selected after implantation for achieving an effective operation of the implantable medical device 1, implantation may become easy. In particular, it is not required to place a particular electrode 132A-132D at a particular location and depth within the tissue in order to for example reach the left bundle branch LBB. The implantable medical device 1 may generally be placed on the tissue in the vicinity of the left bundle branch LBB, wherein subsequently - after implantation - a subset of the electrodes 132A-132D may be selected for emitting and/or sensing electrical signals.

The implantable medical device 1 may comprise more than one anchoring element 13, 14, for example two, three, four, five, six or even more anchoring elements 13, for example in the shape of tines. Each anchoring element 13, 14 herein may comprise one or multiple electrodes 132A-132D being placed at different locations and the respective anchoring element 13, 14.

In the embodiments of Figs. 4, 5 and 8, a further electrode 152 is placed on the body 150 of the implantable medical device 1, the further electrode 152 being arranged outside of tissue when the device is anchored at a location of interest. The further electrode 152 may for example have a ring shape of a coil shape and may e.g. serve as a shock electrode to provide for a defibrillation function.

The idea of the invention is not limited to the embodiments described above.

The implantable medical device may have the shape of a stimulation device comprising leads, or may have the shape of a leadless pacemaker device.

### List of Reference Numerals

- 1: Implantable medical device
- 10: Lead
- 100: Lead body
- 101: Distal end
- 11: Lead
- 12: Generator
- 13: Anchoring element
- 130, 131: End
- 132A-D: Electrode
- 133A-C: Insulating layer
- 134A-C: Conductive layer
- 135: Secondary anchoring member
- 136: Element body
- 137: Conductor
- 14: Anchoring element
- 140, 141: End
- 15: Leadless device
- 150: Body (housing)
- 151: Distal end
- 152: Electrode
- 16: Processing device
- AVN: Atrioventricular node
- E: Length of extension
- H: HIS bundle
- H1-H4: Height
- L: Longitudinal axis
- LA: Left atrium
- LBB: Left bundle branch
- LV: Left ventricle
- M: Intra-cardiac tissue (myocardium)
- RA: Right atrium
- RBB: Right bundle branch
- RV: Right ventricle
- SAN: Sinoatrial node
- V: Superior vena

## Claims

1. An implantable medical device (1) for implantation into a patient, comprising:
a body (100, 150), and
an anchoring element (13) for anchoring the body (100, 150) to tissue at a location of interest, the anchoring element (13) being arranged on the body (100, 150) and having an electrode arrangement for at least on of emitting an electrical stimulation signal and sensing an electrical sense signal,
**characterized in that** the electrode arrangement comprises a multiplicity of electrodes (132A-132D) arranged at different locations on the anchoring element (13).

2. The implantable medical device (1) according to claim 1, **characterized in that** the body (100, 150) forms a distal end (101, 151) to be placed on tissue upon implantation of the implantable medical device (1), the anchoring element (13) being arranged on and extending from the distal end (101, 151).

3. The implantable medical device (1) according to claim 1 or 2, **characterized in that** the body (100) is formed by a lead (10) which is connectable to a generator (12) of the implantable medical device (1).

4. The implantable medical device (1) according to claim 1 or 2, **characterized in that** the body (150) is formed by a housing of a leadless pacemaker device (15).

5. The implantable medical device (1) according to one of the preceding claims, **characterized in that** the anchoring element (13) extends from the body (100, 150) along an axial length of extension (E), wherein the electrodes (132A-132D) are axially displaced with respect to one another along the axial length of extension (E).

6. The implantable medical device (1) according to one of the preceding claims, **characterized in that** at least some of the electrodes (132A-132D) are arranged at different heights with respect to the body (100, 150).

7. The implantable medical device (1) according to one of the preceding claims, **characterized in that** the anchoring element (13) is formed by a helically wound coil element, a linearly extending spike element, or a flexibly bendable tine.

8. The implantable medical device (1) according to one of the preceding claims, **characterized in that** the anchoring element (13) comprises at least one secondary anchoring member (135) configured to anchor to tissue.

9. The implantable medical device (1) according to one of the preceding claims, **characterized in that** at least some of the electrodes (132A-132D) are electrically separated from one another.

10. The implantable medical device (1) according to one of the preceding claims, **characterized in that** the anchoring element (13) comprises a first electrically conductive layer (134A) forming a first electrode (132A), a second electrically conductive layer (134B) forming a second electrode (132B) and an electrically insulating layer (133B) electrically separating the first electrically conductive layer (134A) and the second electrically conductive layer (134B).

11. The implantable medical device (1) according to one of the preceding claims, **characterized in that** the anchoring element (13) comprises an element body (136) to which the multiplicity of electrodes (132A-132D) is attached.

12. The implantable medical device (1) according to one of the preceding claims, **characterized by** a processing device (16) configured to control said electrodes (132A-132D) for at least one of emitting an electrical stimulation signal and sensing an electrical sense signal using at least one of said electrodes (132A-132D).

13. The implantable medical device (1) according to one of the preceding claims, **characterized in that** the body (100, 150) comprises a further electrode (152) placed at a distance from the anchoring element (13).

14. The implantable medical device (1) according to claim 13, **characterized in that** the further electrode (152) is formed as a ring electrode or as a coil electrode extending about the body (100, 150).
